# FASCICULE DE BREVET EUROPEEN

(11) **EP 3 014 162 B1**
(45) Date de publication et mention de la délivrance du brevet: **08.08.2018**
(21) Numéro de dépôt: 14741346.2
(22) Date de dépôt: 25.06.2014
(51) Int. Cl.: F16L 37/098, F16L 33/025, A61J 1/14, A61M 39/10, A61M 39/18

(54) **CONNECTEUR FLUIDIQUE ASEXUÉ AVEC COLLIER ET PROTECTION**
NEUTRALER FLUIDSTECKER MIT KLAMMER UND SCHUTZ
ASEXUAL FLUID CONNECTOR HAVING A CLAMP AND PROTECTION

(30) Priorité: 28.06.2013 FR 1356353
(43) Date de publication de la demande: 04.05.2016
(73) Titulaire: SARTORIUS STEDIM FMT SAS, 13400 Aubagne (FR)
(72) Inventeur: BLAKE, Florian, F-83400 Hyères (FR); GIBELIN, Jérémy, F-83330 Le Beausset (FR)
(74) Mandataire: Derambure Conseil
(86) Numéro de dépôt international: PCT/FR2014/051608
(87) Numéro de publication internationale: WO 2014/207385

(56) Documents cités:
- US-A1- 2003 030 272
- US-A1- 2006 192 165
- US-A1- 2007 001 453
- US-A1- 2009 208 277
- US-A1- 2010 133 807
- US-A1- 2010 230 950
- US-A1- 2012 227 221

## Description

L'invention concerne les connexions fluidiques, en particulier les dispositifs de connexion ou raccordement fluidiques permettant de raccorder une conduite fluide à une autre conduite ou à un récipient, dans le domaine des applications biopharmaceutiques.

Plus précisément, les conduites ou tubes utilisés dans le domaine biopharmaceutique sont des tubes souples, voire très souples, qui servent à transporter des substances biopharmaceutiques diverses, avec le plus souvent des précautions d'asepsie nécessaires.

Les dispositifs de connexions fluidiques comprennent très couramment un premier connecteur de type mâle (c'est-à-dire formant une interface mâle) apte à être reçu dans un second connecteur complémentaire formant une interface femelle, ce qui est une solution simple bien maîtrisée. Toutefois, on ne peut pas raccorder un connecteur de type mâle sur une autre de type mâle, ni un connecteur de type femelle sur un autre type femelle.
Dans le cadre de l'assemblage d'un tube souple sur un autre tube ou un contenant au moyen d'une connexion fluidique, on veut améliorer l'interopérabilité des connecteurs, pour faciliter la constitution d'ensembles modulaires biopharmaceutiques.
On connaît déjà des connexions fluidiques permettant de raccorder ensemble deux connecteurs dits « asexués » (en anglais 'genderless') qui sont fonctionnellement équivalents du point de vue du couplage. Cependant, les connecteurs asexués fluidiques connus nécessitent généralement un mouvement d'insertion axial suivi d'un mouvement de rotation autour de l'axe.

De plus, les précautions de stérilité ou d'asepsie de telles applications rendent nécessaire un bon contrôle des connexions fluides qui sont réalisées entre différentes entités d'un ensemble biopharmaceutique tels que tubes, poches, filtres, etc....
On cherchera donc des moyens de vérifier simplement et de façon fiable la bonne position de couplage, c'est-à-dire que le mouvement d'accouplement a atteint la position finale correcte, cette dernière étant le cas échéant sécurisée par des moyens de verrouillage.

Pour les connecteurs asexués à mouvement de translation axiale suivi de rotation, la bonne réalisation de la rotation est difficile à vérifier.

Il est donc apparu un premier besoin de proposer un dispositif de connexion permettant de raccorder ensemble deux connecteurs asexués avec un mouvement de couplage uniquement de type translation axiale.

Dans les applications biopharmaceutiques, les tubes souples permettent la circulation, le passage, la communication d'un fluide, tel qu'un fluide biopharmaceutique et peut être raccordé soit à un tube souple similaire soit à un récipient ou un contenant, qui peut être rigide ou souple lui aussi.

Le récipient ou contenant en question peut être en l'espèce un récipient de stockage et/ou de traitement d'un contenu tel qu'un produit biopharmaceutique. Un tel récipient s'entend en l'espèce d'un récipient rigide ou semi rigide réutilisable ou d'un récipient souple à usage unique tel qu'une poche, voire une cartouche filtrante.
Cette poche peut être une poche dite 2D, substantiellement peu épaisse, telle que celle commercialisée par Sartorius Stedim Biotech sous la marque Flexboy®, dont le volume, typiquement, peut être compris entre 50 millilitres et 50 litres. Cette poche souple peut aussi être une poche dite 3D telle que celle commercialisée par Sartorius Stedim Biotech sous la marque Flexel®, avec un plus grand volume et une taille substantielle dans les trois dimensions. Il faut noter qu'un tube tel que celui auquel s'applique l'invention peut être interposé entre deux poches ou un plus grand nombre de poches.

Un tube tel que celui auquel s'applique l'invention, habituellement de section circulaire, est réalisé typiquement en matière plastique telle que le silicone, les élastomères thermoplastiques (TPE), mais également le PVC, la liste n'étant pas limitative. Il présente une certaine tenue d'ensemble et, simultanément, à la fois une certaine flexibilité d'ensemble et une certaine flexibilité locale, ce qui permet, moyennant l'exercice d'une force suffisante, de pincer le tube ou de le déformer substantiellement radialement.

Dans une réalisation typique, par exemple, le tube a un diamètre extérieur compris entre par exemple 8 millimètres et 30 millimètres, l'épaisseur dépendant du matériau, du diamètre et des applications.

Dans l'art connu, pour accoupler un tel tube souple, on enfile celui-ci sur un embout tubulaire, après quoi on place un collier de serrage autour du tube, puis on procède au serrage du collier. Ainsi, le collier serré exerce une pression radiale vers l'intérieur pour maintenir le tube souple sur l'embout, d'une part pour assurer une bonne étanchéité du tube vis-à-vis de l'embout et d'autre part pour éviter qu'une traction sur le tube ne conduise à un désengagement du tube de l'embout.

S'agissant de tels colliers de serrage, on peut utiliser par exemple un collier en matière plastique, de type polyamide par exemple en Rilsan®. Ce type de collier plastique, aussi parfois appelé Serflex®, comprend un système de crans sur une bande qui coopèrent avec un crochet à verrouillage agencé dans la tête, de manière à ce que le serrage ne soit pas réversible. En d'autres termes, une fois que l'on a engagé la bande dans la tête pour former une boucle, on tire sur la bande pour réduire le diamètre de la boucle et serrer le collier, le retour en arrière étant empêché par le crochet à verrouillage en prise dans un des crans de la bande. Après serrage, pour éviter que la bande ne dépasse trop du diamètre de la boucle du collier, on sectionne la portion libre de bande à proximité de la tête du collier. La partie restante non détachée de la bande présente souvent une arête vive qui peut s'avérer tranchante.

En alternative au collier plastique, on peut aussi utiliser un collier métallique qui se présente sous la forme d'un anneau préformé muni d'une ou deux oreilles faisant saillie vers l'extérieur relativement à la forme générale de l'anneau du collier, ce type de collier est parfois appelé collier Oetiker®. Après insertion du collier sur le tube à maintenir, on procède au moyen d'un outil au pincement de l'oreille (ou des oreilles) du collier ce qui provoque une déformation rémanente et ainsi un rétrécissement du diamètre principal de l'anneau et par conséquent serrage du collier sur le tube. Ce type de serrage par anneau métallique est particulièrement robuste et fiable. Toutefois, à l'endroit de l'oreille pincée par l'outil, il peut se présenter une aspérité ou une bavure qui forme une arête vive qui peut s'avérer agressive.

Lorsque de tels colliers, qu'ils soient plastiques ou métalliques, sont installés dans des ensembles biopharmaceutiques, ces derniers peuvent être amenés à être transportés ou déplacés, et par conséquent il existe un risque d'agression par des parties agressives de ces colliers vis-à-vis d'autres éléments de l'ensemble biopharmaceutique, en particulier des poches souples ou des tubes souples, ce qui peut entraîner une fuite ou une perte de stérilisation préjudiciable à l'application biopharmaceutique.

De plus, ces colliers sont faciles d'accès (et donc peuvent être démontés) et ne permettent pas de garantir une image et une esthétique satisfaisantes.

Il existe par conséquent un second besoin d'éviter que le collier de serrage ne puisse représenter un danger pour les éléments environnants. Le document US 2010/0133807 A1 divulgue un dispositif de connexion fluidique selon le préambule de la revendication 1.

Le document US 2003/0030272 A1 divulgue un autre dispositif de connexion fluidique.

Ci-après, un exposé de l'invention telle que caractérisée dans les revendications pour proposer une amélioration destinée à pallier, au moins en partie, un des inconvénients précités de l'art antérieur connu. Selon un premier aspect, l'invention a pour objet un dispositif de connexion fluidique selon la revendication 1. Moyennant quoi, il est ainsi proposé un dispositif de connexion fluidique à base de connecteurs asexués, ce qui favorise l'interopérabilité, la modularité et la composition d'ensembles biopharmaceutiques modulaires car tous les connecteurs asexués ainsi formés peuvent être raccordés avec n'importe quel autre connecteur du même type asexué, sans qu'il soit besoin de se préoccuper du type mâle ou femelle de chaque connecteur à raccorder. En outre, il n'y a pas besoin d'effectuer une opération de rotation sur la connexion après le mouvement de translation axiale, ce qui facilite la vérification visuelle de la bonne position d'accouplement. Selon l'invention, les pattes de clipsage d'un des deux connecteurs sont venues de matière à partir du corps de ce connecteur et chacune des pattes de clipsage passe en position de couplage au travers d'une fente ménagée dans une collerette de l'autre connecteur. Ainsi, on propose une collerette qui facilite la préhension et le raccordement manuel du dispositif, compatible avec l'agencement compact des moyens de clipsage, dans un connecteur de type asexué. Selon l'invention, les pattes de clipsage s'écartent vers l'extérieur pendant le mouvement d'accouplement et sont rappelées vers leur position de repos en fin de mouvement d'accouplement pour venir porter sur les surfaces d'arrêt ; de sorte que l'opérateur qui réalise l'accouplement bénéficie d'un retour visuel et/ou auditif et/ou haptique du bon accouplement des connecteurs. Selon l'invention, chaque connecteur comprend un corps sensiblement cylindrique et les pattes de clipsage s'étendent, en position de couplage, de façon adjacente au corps du connecteur opposé et à l'extérieur de ce dernier, et sont rappelées élastiquement radialement vers l'intérieur si on tente de les écarter de leur position de repos, de sorte qu'il faut les écarter toutes simultanément pour déverrouiller la connexion. Moyennant quoi l'encombrement radial du dispositif de verrouillage pour ce type de connecteurs asexués est tout à fait optimisé, et de plus le verrouillage est particulièrement robuste.

Selon une réalisation, chaque patte de clipsage présente une forme d'anse. Ce qui procure une robustesse mécanique satisfaisante aux pattes de clipsage.

Selon l'invention, le dispositif peut comprendre en outre **un capot de protection** formé par **deux demi-portions** configurées pour se refermer l'une sur l'autre dans la direction radiale au moins partiellement autour du premier connecteur et du deuxième connecteur, le capot formant témoin de bon couplage dans sa position fermée et clipsée. Moyennant quoi, la position correcte du capot de protection permet d'indiquer le bon couplage des premier et second connecteurs.

Selon une réalisation, le capot de protection comprend deux portions généralement hémicylindriques complémentaires reliées par une portion souple de charnière, et les deux portions sont aptes à être clipsées l'une à l'autre dans une zone diamétralement opposée à la zone de charnière. De sorte que l'on peut installer facilement le capot de protection autour des premier et second connecteurs, en particulier autour de leurs collerettes respectives jointes. De plus le capot de protection peut être réalisé avantageusement en une seule pièce moulée en matière synthétique.

Selon une réalisation, le capot comprend une rainure interne annulaire apte à former, en position de couplage, un verrouillage immobilisant les collerettes adjacentes appartenant respectivement aux premier et deuxième connecteurs. Moyennant quoi, la position correcte du capot de protection indique de façon fiable le couplage et verrouillage des premier et second connecteurs.

Selon une réalisation, la rainure interne annulaire forme au moins une portion tronconique (de section de forme trapézoïdale) de manière à exercer une pression axiale sur les collerettes, ce qui tend à rapprocher les deux connecteurs. De sorte que l'on bénéficie du mouvement de clipsage du capot de protection pour terminer le cas échéant la course d'accouplement des premier et second connecteurs.

Selon une réalisation, en position de couplage, les deux connecteurs sont disposés symétriquement par rapport au plan de joint avec en outre un décalage angulaire de 360°/2N. Ceci permet de fournir une interface asexuée simple avec plusieurs positions angulaires possibles de couplage, en l'occurrence N positions angulaires possibles.

Selon une réalisation, le nombre N est compris entre 1 et 10, de préférence égal à 4 ; moyennant quoi le nombre de positions angulaires possibles pour l'accouplement est tel que cela facilite la réalisation de l'accouplement.

Selon une réalisation, chaque connecteur comprend en outre un joint d'étanchéité disposé radialement intérieurement dans le corps cylindrique, les deux joints d'étanchéité étant mis en pression dans le sens axial lorsque les deux connecteurs sont en position de clipsage. Ceci représente une solution standard bien maîtrisée pour la fonction étanchéité de connecteurs asexués.

Selon une réalisation, chaque connecteur comprend en outre une membrane aseptique de fermeture provisoire agencée sur la face avant de chacun des joints d'étanchéité et destinée à être retirée après couplage des connecteurs, de manière à pouvoir mettre en communication les deux espaces fluidiques sans mise en communication avec l'air environnant ; de sorte que l'on peut réaliser des ensembles biopharmaceutiques modulaires en respectant les conditions de stérilité ou de protection vis-à-vis de l'air ambiant.

Selon une réalisation, la fermeture du capot provoque, après le retrait de des membranes de fermeture, une sur-course de couplage qui accentue la pression axiale entre les joints d'étanchéité ; moyennant quoi la pression s'exerçant sur les joints d'étanchéité est accrue et la qualité de l'étanchéité est améliorée.

Selon l'invention, le dispositif de connexion comprend en outre un collier de serrage apte et destiné à être disposé autour de l'extrémité du premier tube pour serrer ledit tube sur un embout tubulaire du premier connecteur, et dans lequel le capot de protection comprend au moins une extension tubulaire apte à être positionnée, le capot étant clipsé en position clipsée, au moins partiellement en vis-à-vis radialement du collier de serrage, moyennant quoi l'extension tubulaire évite que le collier de serrage ne puisse entrer en contact directement avec des éléments externes. Moyennant quoi le collier de serrage ne peut pas entrer en contact directement avec des éléments externes et on évite ainsi un endommagement possible des poches souples ou des tubes disposés au voisinage immédiat par une partie agressive du collier de serrage.

Selon une réalisation, le collier de serrage est un collier métallique en forme générale d'anneau avec au moins une oreille, ladite oreille étant destinée à être pincée pour provoquer le serrage du collier, et dans lequel l'extension tubulaire est à distance de la surface extérieure du tube. La solution de l'oreille pincée représente une solution standard bien maîtrisée pour la fonction collier de serrage. En outre, plusieurs diamètres différents de tube souple et d'embout tubulaire sont compatibles avec une seule définition de l'extension tubulaire du capot de protection.

Selon une réalisation, un des connecteurs ou le capot de protection peut comprendre en outre un identifiant de type code barre ou étiquette RFID ou encore code couleur. Moyennant quoi l'accès à des informations relatives à la poche souple et/ou au produit biopharmaceutique y contenu est aisé et facilite le processus de traçabilité. Selon une réalisation, les pattes de clipsage des connecteurs comprennent chacune une extension longitudinale faisant saillie vers l'avant pour venir, de manière à fournir une zone de préhension permettant d'écarter les pattes de clipsage afin de déverrouiller la position de couplage ; de sorte que l'on propose une solution pour le déverrouillage du couplage des premier et second connecteurs.

Selon une réalisation, les pattes de clipsage du second connecteur comprennent chacune une extension longitudinale faisant saillie vers l'avant pour venir, en position de couplage recouvrir, dans la direction radiale, le collier de serrage ; moyennant quoi les extensions évitent que le collier ne puisse endommager des éléments externes situés au voisinage immédiat, être sans l'adjonction d'un capot formant pièce distincte.

Selon un deuxième aspect, l'invention a pour objet un ensemble biopharmaceutique comprenant un dispositif de connexion fluidique tel que décrit ci-dessus.

Selon un troisième aspect, l'invention vise aussi une méthode pour former un dispositif de connexion selon la revendication 14. On décrit maintenant brièvement les figures des dessins.
La figure 1 est une vue éclatée du dispositif de connexion conforme à la présente invention.
La figure 2 est une vue en coupe axiale du dispositif de connexion de la figure 1, en position accouplée, selon la ligne de coupe II-II visible sur la figure 3.
La figure 3 est une coupe transversale détaillée du dispositif de connexion de la figure 1, en position accouplée, selon la ligne de coupe III-III visible sur la figure 2.
La figure 4 est une vue en perspective du dispositif de connexion de la figure 1, en position assemblée, représentée sans capot de protection.
La figure 5 représente un autre mode de réalisation, vue en coupe axiale, dans laquelle le second connecteur comprend une embase fixée à une enceinte biopharmaceutique.
La figure 6 illustre une variante de réalisation dans laquelle les pattes de clipsage comprennent une extension axiale en surlongueur.
Les figures 7A-7C représentent une variante de réalisation, avec des membranes de fermeture aseptique provisoires permettant d'opérer le raccordement des connecteurs sans mise à l'air libre.
Ci-après un exposé détaillé de plusieurs modes de réalisation de l'invention assorti d'exemples et de référence aux dessins.

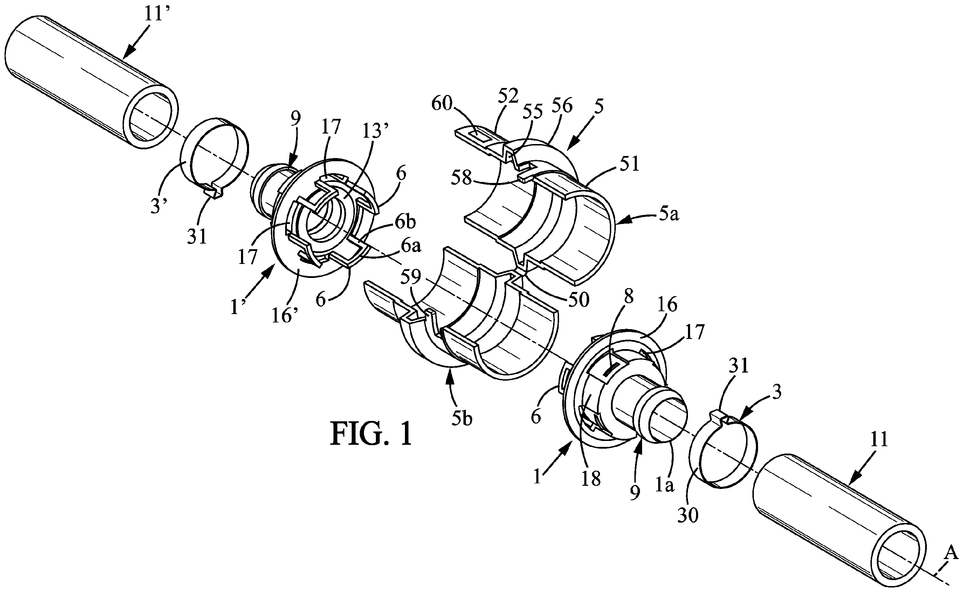

Dans l'exemple illustré aux figures 1 à 4, il s'agit de raccorder premier tube souple **11** à un second tube souple **11'** dans un ensemble biopharmaceutique, au moyen d'un dispositif de connexion fluidique **10** qui comprend un premier connecteur **1** asexué et un second connecteur **1'** asexué, pouvant être accouplés.

Le tube souple 11 peut être défini généralement comme une première paroi 11 délimitant un premier espace fluidique 71. De même, le second tube souple 11' peut être défini généralement comme une seconde paroi 11' délimitant un second espace fluidique 72.

Le premier connecteur **1** est réalisé en matière synthétique, plus précisément il peut être obtenu par moulage d'une matière plastique, par exemple polypropylène, du polyéthylène, du polycarbonate, du polysulfone.
Le premier connecteur 1 comprend un embout tubulaire **9** à une de ses extrémités **1a,** et une interface de couplage avec le second connecteur à l'autre de ses extrémités. L'embout tubulaire 9 est de révolution autour de l'axe A.

L'interface de couplage est une interface de type asexuée qui est prévue pour être insérée dans une interface identique ou similaire de type asexué. Ainsi il n'y a ni entité mâle ni entité femelle dans une telle connexion asexuée.

Le premier connecteur comprend en outre une portion intermédiaire qui se présente sous la forme d'un corps cylindrique 18 centré sur l'axe A. Une collerette **16** s'étend à partir de ce corps cylindrique 18 radialement vers l'extérieur. Dans cette collerette, sont ménagés une pluralité de fentes **17** équitablement réparties autour de la circonférence du corps cylindrique 18. Dans l'exemple illustré, il se trouve 4 fentes en forme d'arc d'environ 45° d'arc, ces arcs autorisent le passage d'un élément au travers de la collerette selon une direction axiale comme il sera vu plus loin.
À l'intérieur du corps cylindrique 18 se trouve un joint d'étanchéité **13** compressible ; ce joint se présente sous la forme d'un anneau centré sur A et de section générale rectangulaire dans l'exemple illustré. Il comprend en particulier une face d'appui 13a, destinée à coopérer avec une autre face d'appui appartenant au joint d'étanchéité d'un autre connecteur opposé auquel le premier connecteur peut être accouplé. Le joint d'étanchéité 13 est de préférence réalisé en matériau élastomère ou silicone.
La face frontale **16a** de la collerette 16, c'est-à-dire celle qui se trouve à l'opposé de la position de l'embout tubulaire, et voisine ou adjacente à un plan de joint **P,** lequel plan de joint définit une position de référence pour l'accostage entre les faces d'appui 13a des joints d'étanchéité de deux connecteurs en position accouplée.
En saillie au-delà du plan de joint, à l'opposé de la position de l'embout tubulaire, se trouvent des pattes de clipsage 6 qui dans l'exemple illustré prennent la forme d'anse de section carrée. La patte de clipsage présente deux portions longitudinales **6b** s'étendant parallèlement à l'axe A à partir de la collerette et une portion transversale **6a** en forme d'arc reliant les extrémités des deux portions longitudinales 6b.

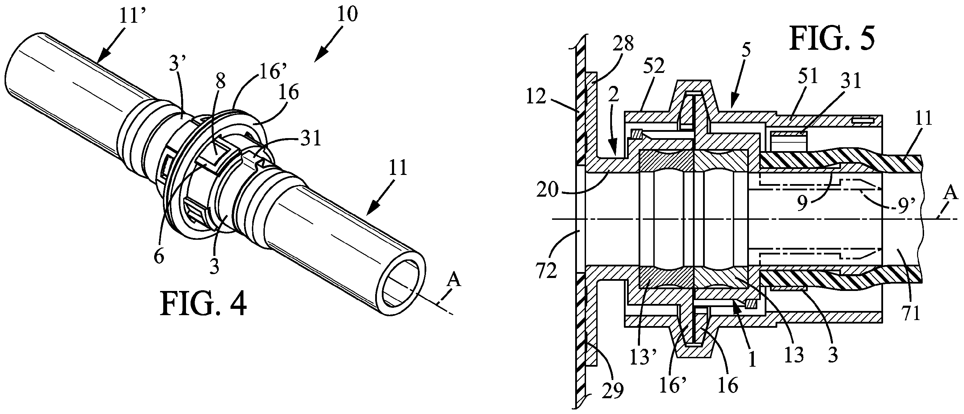

Dans l'exemple illustré, il y a quatre pattes de clipsage **6** qui sont agencées en alternance des fentes 17 déjà décrites et sur le même diamètre que les fentes 17 déjà décrites, chaque patte de clipsage occupe un arc d'environ 45°.
Chaque patte de clipsage 6 est prévue pour passer au travers de la fente correspondante du connecteur opposé, sachant que pour accoupler un premier connecteur sur un second connecteur 1' identique ou analogue, ces derniers doivent présenter un décalage angulaire de sorte que la patte de clipsage du premier se retrouve en face d'une fente du second et vice versa (cf Figs. 1 et 4).

De plus, il est prévu sur le corps cylindrique **18** du connecteur, en retrait par rapport au plan de joint et la collerette, des surfaces d'arrêt **8** qui peuvent se présenter comme des épaulements, lesdites surfaces d'arrêt étant configurées pour coopérer chacune avec une branche transversale 6a d'une patte de clipsage, ceci dans la position d'accouplement, pour procurer ainsi un effet de clipsage, autrement dit de verrouillage, de la position accouplée, comme ceci ressort de la figure 4.
On remarque que les pattes de clipsage 6 sont adjacentes au corps cylindrique 18 du connecteur opposé 6 ; les pattes de clipsage 6 sont agencées à l'extérieur du corps cylindrique 18 du connecteur opposé, et épousent sensiblement la forme extérieure du corps cylindrique.
Chaque surface d'arrêt 8 peut être formée dans une projection en saillie du corps cylindrique, comprenant d'un côté antérieur une rampe douce permettant d'écarter les pattes de clipsage pendant l'insertion du connecteur et du côté postérieur une surface radiale formant la surface d'arrêt 8 sur laquelle vient porter la portion transversale 6a de la patte de clipsage.
Sur la figure 4, en position de couplage, les collerettes 16,16' sont quasiment adjacentes l'une à l'autre lorsque le la portion transversale 6a des pattes bute sur les surfaces d'arrêt 8 sous l'effet de la réaction du joint d'étanchéité. Il sera vu plus loin que l'espace entre les collerettes en position de couplage, plus ou moins important, pourra être rattrapé pour augmenter la pression entre le joint d'étanchéité.
On note que les pattes de clipsage 6 s'écartent radialement vers l'extérieur pendant le mouvement d'accouplement de manière à ce que chaque portion transversale 6a monte au-dessus de la surface d'arrêt 8 en vis-à-vis, laquelle forme dans l'exemple illustré une rampe à cet effet.

Le second connecteur **1',** sur lequel peut se coupler le premier connecteur, est dans l'exemple illustré aux figures 1 à 4, strictement identique au premier connecteur, en matière et formes à. En particulier il comprend un embout tubulaire 9' sur lequel peut être fixé le second tube souple 11', un corps cylindrique 18 avec une collerette 16', un joint d'étanchéité 13', des pattes de clipsage 6, des fentes 17, des surfaces d'arrêt 8, tous ces éléments étant identiques à ceux déjà décrits pour le premier connecteur. Toutefois, le second connecteur pourrait présenter des différences plus ou moins substantielles pour autant que l'interface couplage avec le premier connecteur reste compatible, en particulier la position des branches transversales des pattes de clipsage et la position des surfaces d'arrêt, et dans une moindre mesure le passage procuré par des fentes.
En particulier, les deux connecteurs pourront être couleurs différentes ce qui facilite la vérification visuelle du bon couplage.

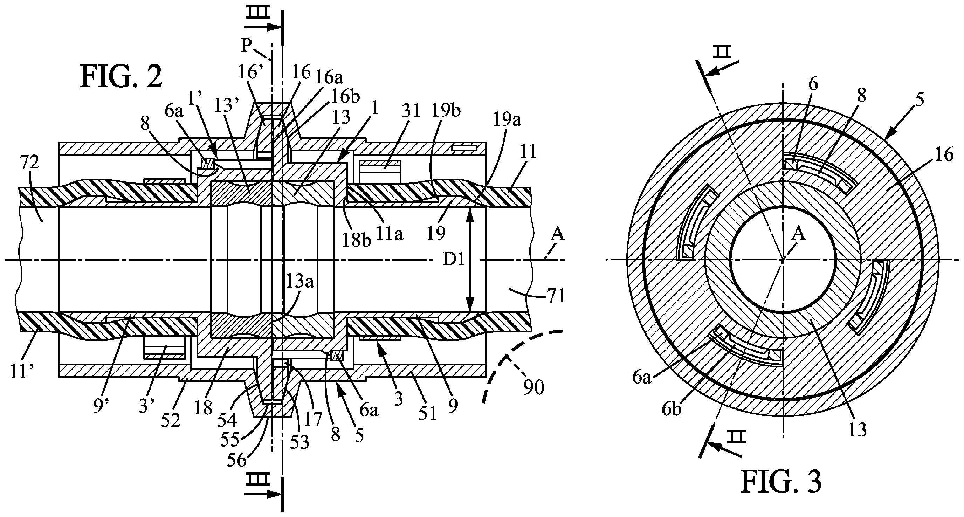

L'embout tubulaire 9 comprend un bourrelet annulaire **19,** qui dans l'exemple illustré présente une rampe douce 19a du côté du tube souple 11 à insérer et un épaulement 19b du côté opposé. L'embout tubulaire pourrait comprendre un nombre plus grand de bourrelets comme par exemple des crans successifs comme connu en soi.
On peut remarquer que le diamètre intérieur D1 de l'embout tubulaire 9 est sensiblement voisin du diamètre intérieur du tube souple 11 au repos.
Lorsque l'on enfile le tube souple 11 sur l'embout tubulaire 9, le tube se déforme radialement vers l'extérieur grâce à la forme de rampe **19a,** puis au fur et à mesure de l'insertion, il retrouve un diamètre plus étroit au niveau de la surface de portée 9a cylindrique.
L'insertion peut se poursuivre jusqu'à ce que l'extrémité avant du tube 11a vienne porter contre la partie postérieure **18b** du corps cylindrique 18 (voir figures 2 et 4).

Une fois que le tube souple est inséré sur l'embout tubulaire 9, on vient placer un collier de serrage **3** autour du tube au niveau de la surface de portée 9a susmentionnée. Il faut noter ici que le collier de serrage 3 peut être disposé préalablement en attente autour d'une partie arrière du tube avant l'opération d'insertion.
Une fois que le collier est dans la position adéquate en vis-à-vis de la surface de portée de l'embout tubulaire, on procède au serrage du collier.
Le collier de serrage représenté aux figures est un collier de type métallique avec une seule oreille **31** prévue pour le serrage. Toutefois il pourrait y avoir plus d'une oreille.

On utilise par exemple une pince pour venir écraser la forme en oreille 31 de manière à diminuer le diamètre de l'anneau **30** formé par le collier de serrage 3. De ce fait, le collier de serrage a alors un diamètre plus petit que celui de la surface extérieure du tube souple au repos, il exerce donc une pression radiale dirigée vers l'intérieur.

Cette pression radiale a deux objectifs : le premier est d'assurer étanchéité suffisamment performante entre le tube 11 et l'embout tubulaire 9, et le deuxième consiste à maintenir mécaniquement le tube autour de l'embout pour éviter qu'une traction exercée sur le tube ne conduise à désengager le tube de l'embout tubulaire, grâce à l'épaulement 19b susvisé.

En outre, il est prévu avantageusement un capot de protection **5** optionnel qui vient recouvrir au moins en partie les premières et secondes collerettes 16,16' appartenant respectivement au premier et second connecteurs 1,1' comme ceci ressort de la figure 2.
Le capot de protection 5 est formé par deux parties **5a,5b** formant deux demi-portions de taille semblable reliées entre elles par une portion de charnière souple **50,** le tout obtenu en une seule opération de moulage. Plus précisément, chacune des parties peut se présenter comme une portion hémicylindrique avec un anneau protubérant 56 en zone centrale bien que d'autres formes soient néanmoins possibles. Les deux portions sont aptes à être clipsées l'une à l'autre dans une zone diamétralement opposée à la zone de charnière, par exemple au moyen de dispositif de crochets de clipsage **58,59,** représentés simplement de façon symbolique sur la figure 1.

Une fois que le capot de protection est installé autour des collerettes des premier et second connecteurs 1,1' , le capot **5** forme ainsi un témoin de bon accouplement des connecteurs. En effet, si les collerettes 16,16' trop éloignés, alors on ne peut pas fermer le capot de protection autour de ces dernières.

Avantageusement, le capot comprend une rainure interne **55** annulaire apte à former, en position de couplage, un verrouillage immobilisant les collerettes 16,16' adjacentes l'une contre l'autre comme ceci ressort de la figure 2.
De plus, cette rainure interne peut comprendre au moins une portion tronconique 53,54 (de section de forme trapézoïdale) de manière à exercer une pression axiale sur les collerettes, ce qui tend à rapprocher les deux connecteurs, et augmente la pression de contact mutuel entre les joints d'étanchéité 13,13'.
En complément, pour faciliter le mouvement de fermeture des deux demi-portions du capot sur les collerettes, la partie postérieure radialement extérieure des collerettes peut comprendre un chanfrein **16b** qui facilite l'accostage des portions tronconiques 53,54.

En outre, le capot de protection 5 peut être tel qu'il forme des éléments de protection vis-à-vis du premier collier de serrage 3 sur le premier connecteur 1 et/ou sur le collier de serrage 3' du second connecteur 1'.
Plus précisément, le capot de protection comprend une première extension axiale 51 du côté du premier connecteur et une seconde extension axiale 52 du côté du second connecteur. Dans l'exemple illustré le capot de protection est ainsi symétrique par rapport au plan de joint P de l'accouplement. Il peut donc être installé de manière quelconque dans un sens ou dans le sens opposé.

La première extension axiale 51 forme ce qui est appelé « des éléments de protection » vis-à-vis du premier collier de serrage 3 : en effet celui-ci, en position de couplage, se retrouve dans la zone intérieure définie par cette extension axiale 51. Moyennant quoi, si lors des manipulations ou déplacements du dispositif de connexion, ce dernier peut venir contacter des éléments externes 90 alors ce n'est pas le collier de serrage qui viendra contacter lesdits éléments externes 90 mais au lieu de cela ce seront le capot de protection 5 ici en l'occurrence l'extension axiale 51 qui viendra contacter le ou les éléments externes 90 (voir figure 2).
Ainsi, tout endommagement par contact avec une partie agressive du collier 3 peut être avantageusement évité.
Les mêmes dispositions et avantages sont obtenus, mutatis mutandis, du côté du second connecteur vis-à-vis du collier de serrage 3'.

Il faut bien noter que les extensions axiales pourraient présenter des formes différentes, il pourrait s'agir d'une pluralité de pattes disjointes réparties sur la circonférence, ou encore une pluralité de parois en forme de portions cylindriques, disjointes et réparties sur la circonférence.

Il faut remarquer que le premier connecteur 1 délimite un premier passage creux destiné à être mis en communication fluidique avec le premier espace 71. De même, le second connecteur délimite un second passage creux, destiné à être mis en communication fluidique avec le second espace 72 (l'intérieur du second tube dans l'exemple illustré).

Dans un autre mode de réalisation représentée à la figure 5, le second connecteur **2** n'est pas destiné à recevoir un tube souple, mais plutôt à être fixé sur un récipient **12** destiné à contenir du fluide biopharmaceutique.

Le récipient peut être défini généralement comme une enceinte souple 12 formée par une seconde paroi délimitant second espace fluidique 72.

Un disque large **28** équipé d'un orifice central est fixé par soudure **29** à la paroi 12 de l'enceinte souple. Une portion tubulaire **20** s'étend depuis le disque large 28 jusqu'au corps cylindrique 18 comprenant les surfaces d'arrêt 8, la collerette 16' et le joint d'étanchéité 13'.

Dans cette réalisation, il n'y a pas de second collier de serrage, et de plus la longueur axiale du second connecteur **2** est plus courte que la longueur axiale du premier connecteur 1. Par conséquent, le capot de protection 5 adapté à cette application se présente sous une forme dissymétrique par rapport au plan de joint P, la seconde extension tubulaire 52 étant plus courte que la première extension tubulaire 51. Toutefois, la forme de la rainure annulaire intérieure 55 est tout à fait similaire à ce qui a été décrit plus haut, ainsi que les moyens de protection vis-à-vis du collier de serrage 3.

Il faut remarquer que la figure 5 illustre qu'il est possible de prévoir plusieurs diamètres d'embouts différents 9,9' pour une seule interface de couplage asexué, ce qui facilite la modularité et la constitution d'ensembles biopharmaceutiques utilisant des diamètres de tube variés.

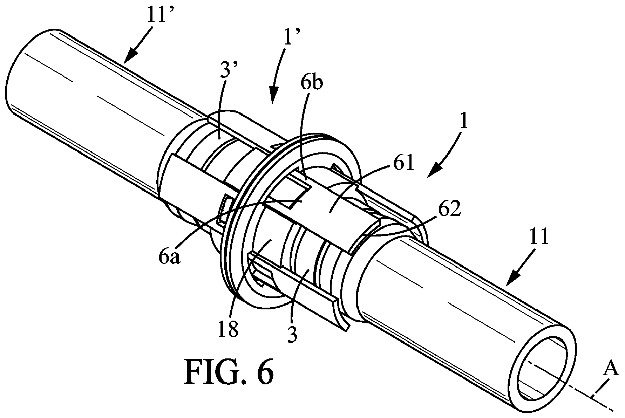

Dans une variante représentée à la figure 6, les pattes de clipsage **6** comprennent chacune une extension axiale **61** qui prolonge la patte vers l'avant, ceci formant une sur-longueur dépassant au-delà du corps du connecteur opposé en position de couplage. Toutes les autres caractéristiques sont identiques ou similaires à ce qui a été décrit précédemment. Il n'y a pas nécessairement besoin de capot de protection dans ce mode. Dans l'exemple illustré, l'extension longitudinale **61** axiale des pattes du second connecteur **1**' dépasse de façon significative du corps **18** du premier connecteur, et par conséquent l'extrémité **62** de cette extension se trouve à distance du tube, ce qui permet de manipuler la patte ou les pattes avec les doigts pour un opérateur.

De plus, ces des extensions axiales vers l'avant forment des éléments de protection vis-à-vis du collier de serrage **3** premier connecteur, empêchant une arête agressive du collier de venir contacter directement un élément externe **90.**

Comme il ressort de la figure 6, de façon symétrique, le premier connecteur **1** comprend aussi des extensions axiales longitudinales des pattes de clipsage, de manière à venir protéger le second collier de serrage **3'.** Cependant, la symétrie n'est pas obligatoire car les extensions axiales des pattes n'interviennent pas directement dans la compatibilité du couplage des connecteurs asexués.

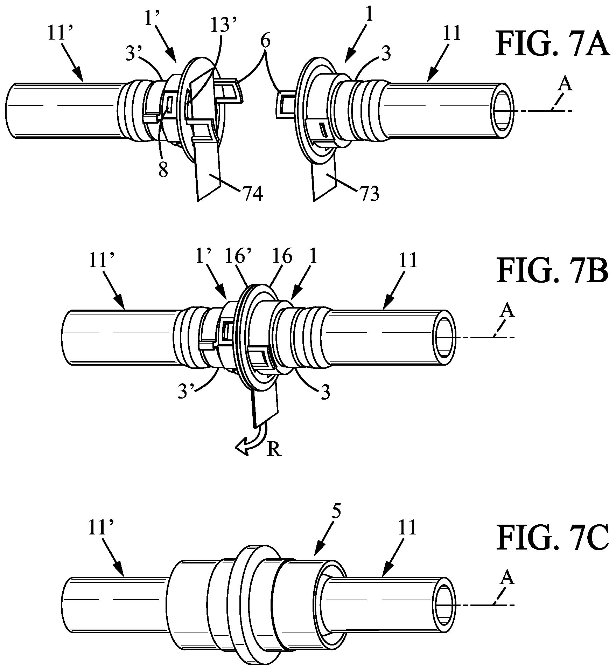

Dans une variante représentée à la figure 7A, chacun des premier et second connecteurs est équipé préalablement à son couplage d'une membrane aseptique de fermeture. Une première membrane **73** est disposée sur la face frontale du joint d'étanchéité **13,** et de façon analogue une seconde membrane **74** est disposée sur la face frontale du joint d'étanchéité **13'** du second connecteur.
Il faut remarquer que dans cette variante, chacun des connecteurs comprend seulement deux pattes de clipsage de manière à laisser assez de place pour l'installation et le retrait de la membrane de fermeture aseptique.
Il est défini une position de clipsage primaire, qui peut être considérée comme une position intermédiaire dans la présente variante de réalisation. Dans cette position de clipsage primaire, la portion transversale **6a** des pattes de clipsage 6 est positionnée en butée sur les surfaces d'arrêt **8,** mais les collerettes 16,16' ne sont pas au contact l'une de l'autre et sont séparées par un espace libre, chaque collerette étant dans cette position agencée légèrement en retrait du plan d'interface **P.**
Tous les autres éléments non décrits à nouveau ici sont supposés être identiques ou similaires à ce qui a été présenté pour le premier mode de réalisation.

Au stade initial du raccordement, les connecteurs **1,1'** sont éloignés et les espaces fluidique **71,72** sont mutuellement isolés et de plus isolés de l'air ambiant.
Pour procéder au raccordement, on réalise tout d'abord un couplage primaire des premier et second connecteurs **1,1',** tel que décrit précédemment selon une manoeuvre similaire à celle décrite pour le premier mode de réalisation. La figure 7B illustre cette position de couplage primaire dans laquelle les membranes aseptiques sont prises en sandwich entre les joints d'étanchéité **13,13'** des deux connecteurs, lesquels exercent l'un contre l'autre une certaine pression axiale.

Puis on procède au retrait des membranes 73,74 de fermeture aseptique, par traction radiale comme illustrée par la flèche **R** de la figure 7B.
À ce moment, les deux espaces fluidiques 71,72 sont mis en communication, et ceci sans qu'ils aient été mis en contact avec de l'air ambiant.
Pour certaines applications, ceci représente une solution suffisante pour le raccordement respectant les conditions d'isolation et de stérilité vis-à-vis de l'air ambiant.

De façon avantageuse, si l'on utilise capot de protection **5** tel que celui présenté précédemment, et comme illustrée à la figure 7C, on met à profit le mouvement de fermeture du capot pour exercer une pression axiale.
Les formes tronconiques de la rainure intérieure annulaire ayant une section trapézoïdale, la fermeture du capot provoque une sur-course axiale de couplage pour rapprocher les collerettes **16,16'** l'une de l'autre et par conséquent accroître la pression de contact entre les deux joints d'étanchéité.

Dans le cas où un capot de protection est utilisé de la sorte, la pression axiale du couplage primaire peut être sensiblement limitée pour faciliter le retrait des membranes de fermeture aseptique.

En outre, il est prévu une caractéristique optionnelle compatible avec toutes les variantes de réalisation précédemment évoquées : il s'agit de l'intégration d'au moins un identifiant **60,** de type code barre ou étiquette électronique par exemple de type RFID. De façon préférée, on dispose cet identifiant cet identifiant sur le premier connecteur, et/ou sur le second connecteur et/ou sur le capot de protection comme illustre à la figure 1. L'identifiant pourrait aussi être une pastille de couleur ou un code couleur de la pièce elle-même.

Il faut noter que selon l'invention, le nombre N de pattes de clipsage peut être un entier positif quelconque de 1 à une dizaine.

## Revendications

1. Dispositif de connexion fluidique (10) apte et destiné à raccorder une première paroi formant un premier tube souple (11) délimitant un premier espace fluidique (71) à une seconde paroi délimitant un second espace fluidique (72) sous forme de deuxième tube souple (11') ou d'enceinte souple (12), semi-rigide ou rigide à usage unique, dans un ensemble biopharmaceutique, comprenant:
- un premier connecteur (1), délimitant un premier passage creux, apte et destiné à être raccordé au premier espace fluidique
- un deuxième connecteur (1';2), analogue au premier connecteur, délimitant un second passage creux, apte et destiné à être raccordé au second espace fluidique,
le premier connecteur et le second connecteur étant aptes et destinés à venir se coupler mutuellement de manière asexuée, selon un mouvement d'insertion essentiellement de translation axiale selon l'axe A, jusque dans une position de couplage relative, laquelle définit un plan de joint (P) perpendiculaire à l'axe principal,
chacun des premier et deuxième connecteurs comprenant, de façon alternée selon une direction circonférentielle, N pattes (6) de clipsage flexibles et N surfaces d'arrêt (8), N étant un nombre entier strictement positif,
les pattes de clipsage flexible faisant saillie axialement vers l'avant par rapport au plan de joint et les surfaces d'arrêt (8) étant arrangées en retrait du plan de joint, de manière à ce que, en position de couplage, les pattes de clipsage de l'un des connecteurs viennent se clipser au delà des surfaces d'arrêt de l'autre des connecteurs, de sorte que la position ainsi obtenue est verrouillée par les pattes de clipsage,
dans lequel les pattes de clipsage d'un des connecteurs sont **venues de matière** à partir du corps (18) de ce connecteur et chacune des pattes de clipsage (6) passe en position de couplage au travers d'une fente (17) ménagée dans une collerette (16) de l'autre connecteur,
dans lequel chaque connecteur comprend un **corps sensiblement cylindrique** et dans lequel les pattes de clipsage s'étendent, en position de couplage, de façon adjacente au corps du connecteur opposé et à l'extérieur de ce dernier, les pattes de clipsage **s'écartent vers l'extérieur** pendant le mouvement d'accouplement, et sont rappelées élastiquement radialement vers l'intérieur, si on tente de les écarter de leur position de repos, de sorte qu'il faut les écarter toutes simultanément pour déverrouiller la connexion, de sorte que la position ainsi obtenue est verrouillée par les pattes de clipsage, **caractérisé par** le dispositif comprenant en outre un capot de protection (5) formé par deux demi-portions (5a,5b) configurées pour se refermer l'une sur l'autre dans la direction radiale au moins partiellement autour du premier connecteur et du deuxième connecteur, le capot formant témoin de bon couplage dans sa position clipsée,
et comprenant en outre un collier de serrage (3) apte et destiné à être disposé autour de l'extrémité du premier tube souple (11) pour serrer ledit tube sur un embout tubulaire (9) du premier connecteur (1), et dans lequel le capot de protection (5) comprend au moins une extension tubulaire (51) apte à être positionnée, capot clipsé en position de couplage, au moins partiellement en vis-à-vis radialement du collier de serrage, moyennant quoi l'extension tubulaire évite que le collier de serrage ne puisse entrer en contact directement avec des éléments externes (90).

2. Dispositif selon la revendication 1, dans lequel chaque patte de clipsage présente une forme d'anse.

3. Dispositif selon la revendication 1 ou 2, dans lequel les deux demi-portions (5a,5b) sont généralement hémicylindriques complémentaires reliées par une portion souple de charnière (50), et les deux portions étant aptes à être clipsées l'une à l'autre dans une zone diamétralement opposée à la zone de charnière.

4. Dispositif selon l'une des revendications précédentes, dans lequel le capot comprend une rainure interne (55) annulaire apte à former, en position de couplage, un verrouillage immobilisant des collerettes (16,16') adjacentes appartenant respectivement aux premier et deuxième connecteurs.

5. Dispositif selon l'une des revendications précédentes, dans lequel la rainure interne (55) annulaire forme au moins une portion tronconique de manière à exercer une pression axiale sur les collerettes, ce qui tend à rapprocher les deux connecteurs.

6. Dispositif selon l'une des revendications précédentes, dans lequel, en position de couplage, les deux connecteurs sont disposés symétriquement par rapport au plan de joint avec en outre un décalage angulaire de 360°/2N.

7. Dispositif selon l'une des revendications précédentes, dans lequel chaque connecteur comprend en outre un joint d'étanchéité (13) disposé radialement intérieurement dans le corps cylindrique (18), les deux joints d'étanchéité (13,13') étant mis en pression dans le sens axial lorsque les deux connecteurs sont en position de clipsage.

8. Dispositif selon la revendication 7, comprenant en outre une membrane aseptique de fermeture provisoire (73,74) agencée sur la face avant de chacune des joints d'étanchéité et destinée à être retirées après couplage des connecteurs, de manière à pouvoir mettre en communication les deux espaces fluidiques (71,72) sans mise en communication avec l'air environnant.

9. Dispositif selon la revendication 8, dans lequel la fermeture du capot provoque, après le retrait de des membranes de fermeture, une surcourse de couplage qui accentue la pression axiale entre les joints d'étanchéité.

10. Dispositif selon l'une des revendications 1-9, dans lequel un des connecteurs ou le capot de protection comprend en outre un identifiant (60) de type code barre ou étiquette RFID ou encore code couleur.

11. Dispositif selon l'une des revendications 1-9, dans lequel les pattes de clipsage des connecteurs comprennent chacune une extension longitudinale (61) faisant saillie vers l'avant de manière à fournir une zone de préhension permettant d'écarter les pattes de clipsage afin de déverrouiller la position de couplage.

12. Dispositif selon l'une des revendications précédentes, dans lequel les pattes de clipsage du second connecteur comprennent chacune une extension longitudinale (61) faisant saillie vers l'avant pour venir, en position de couplage recouvrir, dans la direction radiale, le collier de serrage.

13. Ensemble biopharmaceutique comprenant un dispositif de connexion fluidique selon l'une des revendications précédentes.

14. Méthode pour former un dispositif de connexion destiné à raccorder une première paroi formant un premier tube souple (11) délimitant un premier espace fluidique (71) à une seconde paroi délimitant un second espace fluidique (72) sous forme de deuxième tube souple (11') ou d'enceinte souple (12) ou rigide à usage unique, dans un ensemble biopharmaceutique, la méthode comprenant les étapes :
/a/ fournir un premier connecteur (1) et un second connecteur (1', 2), tous deux à interface asexuée, et équipé chacun d'une collerette (16, 16') et d'un joint d'étanchéité (13, 13') avec une membrane aseptique (73, 74) fermant l'orifice défini par le joint d'étanchéité, et comprenant chacun, de façon alternée selon une direction circonférentielle, N **pattes** (6) **de** clipsage flexibles et N surfaces d'arrêt (8), N étant un nombre entier strictement positif, en disposant en outre un collier de serrage (3) autour de l'extrémité du premier tube souple (11) pour serrer ledit tube sur un embout tubulaire (9) du premier connecteur (1),
/b/ réaliser un couplage de base primaire de premier et second connecteurs selon un mouvement d'insertion essentiellement de translation axiale selon l'axe A, jusque dans une position de couplage relative, laquelle définit un plan de joint (P) perpendiculaire à l'axe principal, les pattes de clipsage flexibles faisant saillie axialement vers l'avant par rapport au plan de joint et les surfaces d'arrêt (8) étant arrangées en retrait du plan de joint, de manière à ce que, en position de couplage, les pattes de clipsage de l'un des connecteurs viennent se clipser au-delà des surfaces d'arrêt de l'autre des connecteurs,
/c/ retirer les membranes de fermeture aseptique (73, 74),
/d/ insérer un capot de protection (5), comprenant une rainure intérieure (55) annulaire ayant une section comprenant deux formes tronconiques (53, 54), la fermeture du capot provoquant une sur course axiale de couplage pour accroître la pression de contact entre les deux joints d'étanchéité (13, 13'), ledit capot de protection étant formé par deux demi-portions (5a,5b) configurées pour se refermer l'une sur l'autre dans la direction radiale au moins partiellement autour du premier connecteur et du deuxième connecteur, ledit capot formant témoin de bon couplage dans sa position clipsée, ledit capot de protection comprenant au moins une extension tubulaire (51) apte à être positionnée, capot clipsé en position de couplage, au moins partiellement en vis-à-vis radialement du collier de serrage (3), moyennant quoi l'extension tubulaire évite que le collier de serrage ne puisse entrer en contact directement avec des éléments externes (90).

## Patentansprüche

1. Fluidverbindungsvorrichtung (10), die geeignet und dafür bestimmt ist, um in einer biopharmazeutischen Anordnung eine erste Wand, die einen ersten weichen Schlauch (11) bildet, der einen ersten Fluidraum (71) begrenzt, mit einer zweiten Wand, die in Form eines zweiten weichen Schlauchs (11') oder weichen, halbsteifen oder steifen einmal verwendbaren Behälters (12) einen zweiten Fluidraum (72) begrenzt, zu verbinden, aufweisend:
- einen ersten Verbinder (1), der einen ersten hohlen Durchlass begrenzt, der geeignet und dafür bestimmt ist, um an den ersten Fluidraum angeschlossen zu werden,
- einen dem ersten Verbinder analogen zweiten Verbinder (1'; 2), der einen zweiten hohlen Durchlass (82) begrenzt, der geeignet und dafür bestimmt ist, um an den zweiten Fluidraum angeschlossen zu werden,
wobei:
der erste Verbinder und der zweite Verbinder geeignet und dafür bestimmt sind, um sich auf eine neutrale Weise miteinander koppeln zu lassen und zwar mit einer Bewegung eines Einsetzens gemäß einer im Wesentlichen axialen Translation entlang der Achse A bis in eine relative Kopplungsposition, die eine zur Hauptachse senkrechte Verbindungsebene (P) definiert,
sowohl der erste als auch der zweite Verbinder entlang einer Umfangsrichtung auf abwechselnde Weise N flexible Klipsklauen (6) und N Arretierungsflächen (8) aufweisen, wobei N eine ganze Zahl größer Null ist,
die flexiblen Klipsklauen bezüglich der Verbindungsebene axial nach vorne vorragen und die Arretierungsflächen (8) von der Verbindungsebene zurückgesetzt angeordnet sind, so dass in Kopplungsposition die Klipsklauen des einen der Verbinder jenseits der Arretierungsflächen des anderen der Verbinder in Klipseingriff kommen, so dass die auf diese Weise erzielte Position durch die Klipsklauen verriegelt ist,
wobei die Klipsklauen eines der Verbinder **integral aus einem Stück Material** mit dem Körper (18) dieses Verbinders gebildet sind und in Kopplungsposition jede der Klipsklauen (6) sich durch einen Schlitz (17) erstreckt, der in einem Kragen (16) des anderen Verbinders bereitgestellt ist,
jeder Verbinder einen **im Wesentlichen zylindrischen Körper** aufweist und in Kopplungsposition die Klipsklauen an dem Körper des anderen Verbinders und an dessen Außenseite anliegen,
die Klipsklauen während der Kopplungsbewegung **sich nach außen spreizen** und sich elastisch radial nach innen zurückstellen, wenn man versucht, sie aus ihrer Ruheposition zu spreizen, so dass man sie alle gleichzeitig spreizen müsste, um die Verbindung zu entriegeln, so dass die auf diese Weise erzielte Position durch die Klipsklauen verriegelt ist,
**dadurch gekennzeichnet, dass** die Vorrichtung ferner aufweist:
eine Schutzkappe (5), die aus zwei Halbteilen (5a, 5b) gebildet ist, die konfiguriert sind, um sich in radialer Richtung mindestens teilweise um den ersten Verbinder und den zweiten Verbinder herum wieder aufeinander zu schließen, wobei die Kappe in ihrer geklipsten Position Indikator einer guten Kopplung ist, und
eine Befestigungsschelle (3), die geeignet und dafür bestimmt ist, um um das Ende des ersten weichen Schlauchs (11) herum angeordnet zu werden, um den Schlauch an einem rohrförmigen Ansatzstück (9) des ersten Verbinders (1) zu befestigen, und wobei die Schutzkappe (5) mindestens einen rohrförmigen Fortsatz (51) aufweist, der geeignet ist, um, wenn die Kappe in Kopplungsposition geklipst ist, mindestens teilweise radial gegenüber der Befestigungsschelle positioniert zu sein, wodurch der rohrförmige Fortsatz verhindert, dass die Befestigungsschelle in direkten Kontakt mit externen Elementen (90) kommen könnte.

2. Vorrichtung nach Anspruch 1, in welcher jede Klipsklaue bügelförmig ist.

3. Vorrichtung nach Anspruch 1 oder 2, in welcher die zwei Halbteile (5a, 5b) allgemein halbzylindrisch komplementär sind und mittels eines biegsamen Scharnierabschnitts (50) miteinander verbunden sind, und die zwei Teile geeignet sind, um in einer der Scharnierzone diametral gegenüberliegenden Zone mittels Klipsen miteinander verbunden zu werden.

4. Vorrichtung nach einem der vorstehenden Ansprüche, in welcher die Kappe eine ringförmige Innenrille (55) aufweist, die geeignet ist, um in Kopplungsposition eine Verriegelung zu bilden, die die aneinander liegenden Kragen (16, 16') des ersten und zweiten Verbinders fixiert.

5. Vorrichtung nach einem der vorstehenden Ansprüche, in welcher die ringförmige Innenrille (55) mindestens einen kegelstumpfförmigen Abschnitt bildet, um damit einen axialen Druck auf die Kragen in Richtung auf eine Annährung der zwei Verbinder auszuüben.

6. Vorrichtung nach einem der vorstehenden Ansprüche, in welcher in Kopplungsposition die zwei Verbinder symmetrisch bezüglich der Verbindungsebene angeordnet sind, mit einem Winkelversatz von 360°/2N.

7. Vorrichtung nach einem der vorstehenden Ansprüche, in welcher jeder Verbinder ferner ein Dichtungselement (13) aufweist, das in dem zylindrischen Körper (18) radial innen angeordnet ist, wobei die zwei Dichtungselemente (13, 13') in axialer Richtung unter Druck gesetzt sind, wenn die zwei Verbinder in Klipsposition sind.

8. Vorrichtung nach Anspruch 7, ferner mit einer aseptischen provisorischen Verschlussmembran (73, 74), die an der Vorderfläche jedes Dichtungselements angeordnet ist und dafür bestimmt ist, um nach Kopplung der Verbinder zurückgezogen zu werden, um so die zwei Fluidräume (71, 72) miteinander in Verbindung bringen zu können, ohne sie mit der Umgebungsluft in Verbindung zu bringen.

9. Vorrichtung nach Anspruch 8, in welcher nach Zurückziehen der Verschlussmembranen das Schließen der Kappe einen Kopplungsüberhub hervorruft, der den axialen Druck zwischen den Dichtungselementen verstärkt.

10. Vorrichtung nach einem der Ansprüche 1 bis 9, in welcher einer der Verbinder oder die Schutzkappe ferner einen Identifikator (60) des Typs Strichcode oder RFID-Etikette oder auch Farbcode aufweist.

11. Vorrichtung nach einem der Ansprüche 1 bis 9, in welcher die Klipsklauen der Verbinder jeweils einen nach vorne vorragenden Längsfortsatz (61) aufweisen, um eine Greifzone bereitzustellen, die erlaubt, die Klipsklauen zu spreizen, um die Kopplungsposition zu entriegeln.

12. Vorrichtung nach einem der vorstehenden Ansprüche, in welcher die Klipsklauen des zweiten Verbinders jeweils einen nach vorne vorragenden Längsfortsatz (61) aufweisen, um in Kopplungsposition die Befestigungsschelle in radialer Richtung zu bedecken.

13. Biopharmazeutische Anordnung, aufweisend eine Fluidverbindungsvorrichtung nach einem der vorstehenden Ansprüche.

14. Verfahren zur Herstellung einer Verbindungsvorrichtung, die dafür bestimmt ist, um in einer biopharmazeutischen Anordnung eine erste Wand, die einen ersten weichen Schlauch (11) bildet, der einen ersten Fluidraum (71) begrenzt, mit einer zweiten Wand, die in Form eines zweiten weichen Schlauchs (11') oder weichen oder steifen einmal verwendbaren Behälters (12) einen zweiten Fluidraum (72) begrenzt, zu verbinden, wobei das Verfahren die folgenden Schritte aufweist:
/a/ Bereitstellen eines ersten Verbinders (1) und eines zweiten Verbinders (1', 2), die beide eine neutrale Anschlussfläche haben und jeweils einen Kragen (16, 16') und ein Dichtungselement (13, 13') mit einer aseptischen Membran (73, 74) aufweisen, die die von dem Dichtungselement begrenzte Öffnung schließt, sowie jeweils in Umfangsrichtung auf eine abwechselnde Weise N flexible Klipsklauen (6) und N Arretierungsflächen (8) aufweisen, wobei N eine ganze Zahl größer Null ist, ferner mit einer um das Ende des ersten weichen Schlauchs (11) angeordneten Schelle (3), um den Schlauch an einem rohrförmigen Ansatzstück (9) des ersten Verbinders zu befestigen,
/b/ Herstellen einer primären Basiskopplung des ersten und zweiten Verbinders mit einer Bewegung eines Einsetzens gemäß einer im Wesentlichen axialen Translation entlang der Achse A bis in eine relative Kopplungsposition, die eine zur Hauptachse senkrechte Verbindungsebene (P) definiert, wobei die flexiblen Klipsklauen bezüglich der Verbindungsebene axial nach vorne vorragen und die Arretierungsflächen (8) zurückgesetzt von der Verbindungsebene angeordnet sind, so dass in Kopplungsposition die Klipsklauen des einen der Verbinder jenseits der Arretierungsflächen des anderen der Verbinder in Klipseingriff kommen,
/c/ Zurückziehen der aseptischen Verschlussmembranen (73, 74),
/d/ Einsetzen einer Schutzkappe (5), die eine ringförmige Innenrille (55) aufweist, mit einem Abschnitt, der zwei Kegelstumpfformen (53, 54) aufweist, wobei das Schließen der Kappe einen axialen Kopplungsüberhub hervorruft, um den Kontaktdruck zwischen den zwei Dichtungselementen (13, 13') zu erhöhen, wobei die Schutzkappe aus zwei Teilen (5a, 5b) gebildet ist, die konfiguriert sind, um sich in radialer Richtung mindestens teilweise um den ersten Verbinder und den zweiten Verbinder herum wieder aufeinander zu schließen, wobei die Kappe in ihrer geklipsten Position Indikator einer guten Kopplung ist, wobei die Schutzkappe mindestens einen rohrförmigen Fortsatz (51) aufweist, der geeignet ist, um, wenn die Kappe in Kopplungsposition geklipst ist, mindestens teilweise radial gegenüber der Befestigungsschelle (3) positioniert zu sein, wodurch der rohrförmige Fortsatz verhindert, dass die Befestigungsschelle in direkten Kontakt mit externen Elementen (90) kommen könnte.

## Claims

1. Fluid connection device (10) adapted and intended for connecting a first wall forming a first flexible pipe (11) defining a first fluid space (71), to a second wall defining a second fluid space (72) in the form of a second flexible pipe (11') or flexible enclosure (12) that is semi-rigid or rigid and disposable, in a biopharmaceutical assembly, comprising:
- a first connector (1) defining a first hollow passage, adapted and intended for connection to the first fluid space,
- a second connector (1'; 2), similar to the first connector, defining a second hollow passage, adapted and intended for connection to the second fluid space,
the first connector and the second connector being adapted and intended to be coupled together in a genderless manner, by an insertion movement that is essentially an axial translation along the axis A, into a relative coupling position, which defines a mating plane (P) perpendicular to the main axis,
each of the first and second connectors comprising, in an alternating manner along the circumferential direction, N flexible snap-fitting tabs (6) and N stop surfaces (8), N being a strictly positive integer,
the flexible snap-fitting tabs projecting axially forwards relative to the mating plane, and the stop surfaces (8) being set back from the mating plane so that, in the coupling position, the snap-fitting tabs of one of the connectors clip into place beyond the stop surfaces of the other of the connectors, so that the resulting position is locked by the snap-fitting tabs,
wherein the snap-fitting tabs of one connector are **formed integrally** from the body (18) of the connector, and each of the snap-fitting tabs (6) moves into the coupling position through a slot (17) formed in a collar (16) of the other connector,
wherein each connector comprises a **substantially cylindrical body** and wherein in the coupling position the snap-fitting tabs lie adjacent to the body of the opposing connector and externally thereto, the snap-fitting tabs **moving apart outwardly** during the coupling movement and being resiliently biased radially inward if someone attempts to move them away from their rest position, such that they must all be moved apart simultaneously in order to unlock the connection, so that the position thus obtained is locked by the snap-fitting tabs,
**characterized by** the device further comprising a protective cover formed by two half-portions (5a, 5b) configured to close together in the radial direction to at least partially surround the first connector and the second connector, the cover serving as indicator of proper coupling when snap-fitted into its closed position,
and further comprising a pipe clamp (3) adapted and intended to be arranged around the end of the first flexible pipe (11) in order to clamp said pipe onto a tubular nozzle (9) of the first connector (1), and wherein the protective cover (5) comprises at least one tubular extension (51) adapted to be positioned, when the cover is clipped into the coupling position, at least partially facing the pipe clamp in the radial direction, whereby the tubular extension prevents the pipe clamp from coming into direct contact with external elements (90).

2. Device according to claim 1, wherein each snap-fitting tab has a stirrup shape.

3. Device according to claim 1 or claim 2, wherein the two half-portions (5a, 5b) are generally complementary and semi-cylindrical, connected by a flexible hinge portion (50), the two portions being adapted to be snap-fitted together in an area diametrically opposite the hinge area.

4. Device according to one of the preceding claims, wherein the cover comprises an annular inner groove (55) adapted to form, in the coupling position, a lock that immobilizes adjacent collars (16, 16') that are respectively part of the first and second connectors.

5. Device according to one of the preceding claims, wherein the annular inner groove (55) forms at least one tapered portion so as to exert axial pressure on the collars, which urges the two connectors closer together.

6. Device according to one of the preceding claims, wherein, in the coupling position, the two connectors are arranged symmetrically relative to the mating plane, furthermore with an angular displacement of 360°/2N.

7. Device according to one of the preceding claims, wherein each connector further comprises a seal (13) arranged radially inward within the cylindrical body (18), pressure being applied to the two seals (13, 13') in the axial direction when the two connectors are snap-fitted into position.

8. Device according to claim 7, further comprising a temporary aseptic sealing membrane (73, 74) arranged on the front face of each of the seals, and intended to be removed after coupling the connectors so that the two fluid spaces (71, 72) are placed in communication without any communication with the surrounding air.

9. Device according to claim 8, wherein the closing of the cover causes, after removal of the sealing membranes, additional travel in the coupling which increases the axial pressure between the seals.

10. Device according to one of claims 1-9, wherein one of the connectors or the protective cover further comprises an identifier (60) such as a barcode or RFID tag or color code.

11. Device according to one of claims 1-9, wherein the snap-fitting tabs of the connectors each comprise a longitudinal extension (61) which projects forward, so as to provide a gripping area for spreading apart the snap-fitting tabs in order to unlock the coupling position.

12. Device according to one of the preceding claims, wherein the snap-fitting tabs of the second connector each comprise a longitudinal extension (61) which projects forward so as to cover, in the coupling position, the pipe clamp in the radial direction.

13. Biopharmaceutical assembly comprising a fluid connection device according to one of the preceding claims.

14. Method for forming a connection device intended for connecting a first wall forming a first flexible pipe (11) defining a first fluid space (71) to a second wall defining a second fluid space (72) in the form of a second flexible pipe (11') or flexible enclosure (12) or rigid and disposable, in a biopharmaceutical assembly, the method comprising the steps of:
/a/ providing a first connector (1) and a second connector (1', 2), both with a genderless interface, and each equipped with a collar (16, 16') and a seal (13, 13') with aseptic membrane (73, 74) closing off the opening defined by the seal, and each comprising, in an alternating manner along a circumferential direction, N flexible snap-fitting tabs (6) and N stop surfaces (8), N being a strictly positive integer, further comprising a pipe clamp (3) around the end of the first flexible pipe (11) in order to clamp said pipe on a tubular nozzle (9) of the first connector (1),
/b/ creating a basic primary coupling of first and second connectors in an insertion movement that is essentially axial translation along the axis A, into a relative coupling position, which defines a mating plane (P) perpendicular to the main axis, the flexible snap-fitting tabs projecting axially forwards relative to the mating plane and the stop surfaces (8) being set back from the mating plane so that, in the coupling position, the snap-fitting tabs of one of the connectors clip into place beyond the stop surfaces of the other connector,
/c/ removing the aseptic sealing membranes (73, 74),
/d/ inserting a protective cover (5) comprising an annular inner groove (55) having a cross-section comprising two tapered shapes (53, 54), the closing of the cover causing additional axial travel in the coupling to increase the contact pressure between the two seals (13, 13'), said protective cover being formed by two half-portions (5a, 5b) configured to close together in the radial direction to at least partially surround the first connector and the second connector, said cover serving as indicator of proper coupling when snap-fitted into its closed position, said protective cover comprising at least one tubular extension (51) adapted to be positioned, when the cover is clipped into the coupling position, at least partially facing the pipe clamp (3) in the radial direction, whereby the tubular extension prevents the pipe clamp from coming into direct contact with external elements (90).
